# EUROPÄISCHE PATENTSCHRIFT

(11) **EP 3 532 645 B1**
(45) Veröffentlichungstag und Bekanntmachung des Hinweises auf die Patenterteilung: **26.06.2024**
(21) Anmeldenummer: 17788099.4
(22) Anmeldetag: 23.09.2017
(51) Int. Cl.: C21D 9/32, C21D 7/06

(54) **VERFAHREN ZUR BEARBEITUNG EINER ZAHNSTANGE UND DANACH BEARBEITETE ZAHNSTANGE**
METHOD FOR MACHINING A RACK AND RACK MACHINED ACCORDING TO SAID METHOD
PROCÉDÉ D'ÉLABORATION D'UNE CRÉMAILLÈRE ET CRÉMAILLÈRE AINSI OBTENUE

(30) Priorität: 28.10.2016 DE 102016012941
(43) Veröffentlichungstag der Anmeldung: 04.09.2019
(73) Patentinhaber: MVO GmbH Metallverarbeitung Ostalb, 73529 Schwäbisch Gmünd (DE)
(72) Erfinder: VIEWEG, Niels, 86356 Neusäß (DE)
(74) Vertreter: Nowack, Linda
(86) Internationale Anmeldenummer: PCT/DE2017/000316
(87) Internationale Veröffentlichungsnummer: WO 2018/077318

(56) Entgegenhaltungen:
- US-A1- 2016 298 203
- DATABASE WPI Week 201401 Thomson Scientific, London, GB; AN 2013-W45656 XP002778776, & JP 2013 241961 A (HINO MOTORS LTD) 5. Dezember 2013 (2013-12-05)
- MOLZEN M S ET AL: "SHOT PEENING AND HEAT TREATMENT REDUCE STRESS", WELDING JOURNAL, AMERICAN WELDING SOCIETY, MIAMI, FL, US, Bd. 80, Nr. 1, Januar 2001 (2001-01), Seiten 38-42, XP001001069, ISSN: 0043-2296

## Beschreibung

### Technisches Gebiet

Die Erfindung betrifft ein Verfahren zur Bearbeitung einer Zahnstange, wie sie beispielsweise in Lenkungssystemen von Fahrzeugen als sogenannte Lenkungszahnstange verwendet wird. Die Erfindung ist auch für Zahnstangen anderer Systeme anwendbar.

### Stand der Technik

Derartige Zahnstangen werden vorrangig aus Stabstählen hergestellt und umfassen mindestens einen der Arbeitsgänge, wie ein sogenanntes Kurzstück ablängen, Verzahnung in einem Abschnitt anbringen, Härten, Richten, Endenbearbeitung der Stirnseiten, Schleifen, Finishing, Rissprüfung, Waschen, um sie dem Einbau in einem Lenkungssystem oder einem anderen System zur Verfügung zu stellen.

Es werden bei der Herstellung von Kraftfahrzeugen für jedes verbaute Teil wie auch eine Lenkungszahnstange ein niedriges Gewicht und eine erhöhte Langzeitfestigkeit hinsichtlich der Wirtschaftlichkeit des Kraftstoffverbrauchs und der Sicherheit bis hin zu einer weitgehend gleichmäßigen Spannungsverteilung im Zahnstangenwerkstück gefordert.

Gemäß den Erfahrungen in der Fachwelt liegt nach dem Anbringen der Verzahnung, dem Härten und Richten von Zahnstangen mindestens im Abschnitt der Verzahnung ein nachteiliger und undefinierbarer Verlauf von Druck- und Zugspannungen vor. Der praktizierende Fachmann bezeichnet diesen auch als "chaotischen" Spannungsverlauf, auf welches Kriterium im Zusammenhang mit der Erfindung zurückzukommen ist.

Es beschreiben DE 102012100279 A1 und DE 102014105780 A1 Lenkungszahnstangen, die nach den Arbeitsgängen, wie Verformen und Wärmebehandlung oder Verzahnen und Richten vor der Einbaufertigkeit eine weitgehend gleichmäßige Spannungsverteilung im Werkstück aufweisen sollen. Derartige Zahnstangen wurden
■ mittels einer angepassten Vorrichtung zum Induktionshärten zur Erzielung einer gleichmäßigen Härtezone bearbeitet oder
■ im Richtprozeß einer Parametrierung unterzogen.

Die aktuell geforderten Parameter einer gleichmäßigen Spannungsverteilung im Zahnstangenwerkstück können damit nicht erfüllt werden.

Weitere Lösungen zur Bearbeitung gattungsgemäßer Zahnstangen betreffend das Problem der Beherrschung von Spannungszuständen zeigen das Folgende:
DE 69312807 T2 offenbart ein konventionelles Verfahren zur Herstellung einer Lenkzahnstange für einen Einsatz in Kraftfahrzeugen mit dem Fräsen eines Teils einer zylindrischen Stange zu einer halbzylindrischen Form mit einer flachen Oberfläche und das weitere Fräsen eines flachen Abschnittes, um auf dessen Oberfläche die Zahnstange zu bilden. Derartige Zahnstangen genügen den derzeitigen Anforderungen wegen ihres hohen Gewichtes und der hohen Kosten nicht. Besonders nachteilig ist das Gefüge zerstörende Fräsen eines Abschnittes der Oberfläche der Lenkzahnstange.

Um Zahnstangen einfacher und billiger herzustellen, wurden Rohre mit geringeren Wanddicken eingesetzt, woraus sich Probleme der Festigkeit ergaben. Denen sollte durch Lösungen z. B. gemäß JP 2247020 begegnet werden.

So wurden nach DE 69312807 T2 Zahnstangen aus einem Metallrohr mit
■ einem ersten Schritt des Anwendens von Druck, um einen Querschnitt herzustellen, auf dem die Zahnstangen gebildet werden sollen,
■ einem zweiten Schritt des Montierens des Rohres zwischen Werkzeugen, die eine komplementäre Beziehung zu den Zahnstangen aufweisen, und des Pressens eines Dornes mit einem halbkreisförmigen Querschnitt in die Bohrung des Metallrohres hinein, und
■ einem weiteren Schritt des Einsetzens einer Kernstange in ein Ende des Metallrohres und der Verwendung einer Strangpresse für das Strangpressen des Rohres um die Kernstange herum, um die Wanddicke des Rohres zu verringern,
technologisch aufwändig hergestellt.

DE 1 12006000619 B4 offenbart eine Lenkungszahnstange für ein Fahrzeug-Zahnstangenlenkungssystem, das Getriebezähne und einen Schaft aufweist, der einen konstanten Außendurchmesser und eine konstante Wandstärke über den größten Teil seiner Länge besitzt. Dazu umfasst das Bearbeitungsverfahren einen Schmiedevorgang an einem rohrförmigen Teil, bei dem ein längliches Teil in die Bohrung eines ersten Bereichs des rohrförmigen Teils eingesetzt ist. Der Schmiedevorgang formt die Getriebezähne auf dem ersten Bereich, wodurch das längliche Teil in dem rohrförmigen Teil gehalten wird. Vor dem Schmiedevorgang wird das rohrförmige Teil hergestellt, indem ein Formvorgang an einem Rohrstück derart durchgeführt wird, dass der Außendurchmesser des ersten Bereichs kleiner als der konstante Außendurchmesser des Schafts und die Wandstärke des ersten Bereichs größer als die konstante Wandstärke des Schafts ist. Mittels Knetvorgang wird der erste Bereich bei dem Knetvorgang axial zusammengedrückt und das längliche Teil in die Bohrung des rohrförmigen Teils vor dem Knetvorgang eingesetzt. Der erste Bereich wird auf das längliche Teil geknetet. Während des Knetvorgangs wird der erste Bereich auf eine entfernbare und wiederverwendbare Knetspindel geknetet, wobei der Außendurchmesser über die Länge des Rohrstücks durch den Formvorgang reduziert wird.

Trotz möglicher Reduzierung des Außendurchmessers würde die fachmännische Weiterverfolgung dieser Lehre erhebliche technologische Kosten erfordern.

Insgesamt vermitteln diese Lösungen dem Fachmann keine Ansätze, um das Problem "chaotischer" oder nachteiliger Spannungszustände technologisch vorteilhaft beherrschen zu können.

In dem Beitrag "Induktives Härten von Lenkstangen für Elektrolenkungen" wurde darüber berichtet, dass die Anforderungen bezüglich Dauerfestigkeit und Verschleißverhalten an Werkstücken weiter steigen, aber aus Gewichtsgründen die Bauteile nicht größer oder schwerer werden dürfen. Demnach sollen das induktive Härten besonders belasteter Stellen von Zahnstangen sowie die Nutzung von entsprechend hochwertigem, induktiv wärmebehandelten Vormaterial Möglichkeiten darstellen, diesen Anforderungen gerecht zu werden (Autoren Dipl.-Wirtsch.-Ing. Dirk M. Schibisch, Dipl.-Ing. Martin Bröcking, elektrowärme international 3-2013).

Eine andere Lösung beschreibt die DE 102012107501 A1, um bei einer Zahnstange für ein Lenkgetriebe die durch Verformung erzeugten Spannungsspitzen und Querschnittsänderungen zu vermeiden. Demnach werden jene Zahnstange und ein antreibendes Lenkritzel derart angeordnet, dass eine Vorspannung des Druckstückes auf die Zahnstange erreicht wird. Damit werden zwar verbesserte Dämpfungseigenschaften im Sinne geringerer Geräuschentwicklungen erzielt, jedoch erhält der Fachmann keine Hinweise auf eine gleichmäßige Spannungsverteilung im Werkstück.

Der darüber hinausgehende Ausblick vermittelt dem Fachmann, dass erhöhten Festigkeitsanforderungen gerecht werdende Lösungen für Zahnstangen wie Lenkzahnstangen nach beispielsweise EP 1 640 467 B1 durch Gefügeveränderungen erreichbar sind. Demnach weisen diese in ihrem Materialgefüge 0,50 bis 0,60 Gew.-% Kohlenstoff, 0,05 bis 0,5 Gew.-% Silizium, 0,2 bis 1,5 Gew.-% Mangan, 0,0005 bis 0,003 Gew.-% Bor, 0,005 bis 0,05 Gew.-% Titan, 0,0005 bis 0,1 Gew.-% Aluminium und 0,002 bis 0,2 Gew.-% Stickstoff sowie ein durch Eisen und zufällige Verunreinigungen gebildeten Rest auf. Regelmäßig werden solche Stahlstangen für Zahnstangen abgeschreckt und getempert, so dass sie wenigstens in einem Abschnitt der Stahlstange in bestimmten Tiefen ein getempertes bainitisches Gefüge und ein getempertes martensitisches Gefüge erhalten. Diese Gefüge schaffen eine Voraussetzung für gewollte Minimierungen des Durchmessers einer Zahnstange. Ein derartiges Potential für eine Gewichtseinsparung über Minimierung des Durchmessers kann jedoch nach dem Anbringen der Verzahnung nicht ausgeschöpft werden, um das Problem des in der Verzahnung vorliegenden "chaotischen" Eigenspannungsverlaufs mit Spannungsspitzen zu lösen, und zwar ohne Gefügeveränderung.

In AT 515352 A1 (DE 102014225995 A1) wird ein Sinterbauteil mit einer Verzahnung, die Zähne mit Zahnfüßen und Zahnflanken umfasst, beschrieben. Sämtliche Zähne und Zahnfüße der Verzahnung weisen eine plasmanitrierte oder plasmanitrocarburierte Schicht und die Zahnfüße eine Zahnfußdauerfestigkeit nach DIN 3990 von mindestens 200 MPa auf. Das Verfahren zur Herstellung dieses Sinterbauteils umfasst die Schritte Pulverpressen, Sintern und Härten. Wenn auch bei derartigen Sinterbauteilen eine hohe Zahnfußfestigkeit erreichbar ist, die Zahnflanken einen maximalen Wert der Druckeigenspannungen in einem Bereich von 200 MPa bis 1500 MPa aufweisen können, eine weitere Verbesserung der Dauerfestigkeit des Sinterbauteils möglich wird und die Gefahr der Rissbildung im Bereich der Zähne reduzierbar ist und diese Sinterbauteile als Zahnstange mit einer Geradverzahnung oder einer Schrägverzahnung ausführbar sind, kann der Fachmann daraus keinen Ansatz für Lösungen zur Bearbeitung gattungsgemäßer Zahnstangen betreffend das Problem der Beherrschung von Spannungszuständen gewinnen. Die dortige Technologie ist auf das Plasmanitrieren bzw. das Plasmanitrocarburieren zur Härtung von Sinterbauteilen ausgerichtet, um z.B. prozessbedingte Verzüge zu vermeiden.

Dem Fachmann ist bekannt, dass Werkstückoberflächen nachbearbeitet werden, um entweder nachteilige Zugeigenspannungen zu eliminieren oder vorteilhafte Druckeigenspannungen in die kritischen Teile durch mechanische und thermische Mittel einzubringen, so durch Kugelstrahlen oder oben genanntes Induktionshärten. Damit können randschichtnahe Werkstoffzustände, wie Eigenspannungen oder Verfestigungen modifiziert werden. Das Kugelstrahlen ist als sogenanntes Verfestigungsstrahlen wie Shot Peening bekannt, wonach in erster Hinsicht die Oberflächenfestigkeit von Werkstücken erhöht werden soll.

Bei durch Härten nachbehandelten Stählen wird gemäß US 2006/0048867 A1 vermittelt, den von Eigenspannungen verursachten Spannungsrissen dadurch begegnen zu können, indem während einer Behandlung mittels Gasstrahl derartige Oberflächen röntgenographisch bestrahlt und dabei die Ausbildung wahlweiser oder gewünschter Eigenspannungen gemessen werden. Diese Lehre basiert jedoch auf technologisch bedingte Gefügeveränderungen.

Nach DE 102013218413 A1 ist bekannt, einen Längslenker und eine Torsionsstange unmittelbar vor dem Verschweißen auf eine Temperatur zwischen 120 und 150°C vorzuwärmen, wobei nach dem Verschweißen Längslenker und Torsionsstange einem Verfestigungsstrahlen unterzogen werden. Durch das Verfestigungsstrahlen soll die Betriebsfestigkeit gesteigert werden.

Zur Herstellung von Kraftfahrzeugfedern gemäß der DE102011055104 B1 wird aus der US 6,544,360 B1 ein Oberflächenbehandlungsverfahren zitiert, durch das eine entsprechend behandelte Oberfläche einen besseren Spannungsverlauf aufzeigt, wenn Kraftfahrzeugfedern erwärmt und anschließend mit einem Verfestigungsstrahlen behandelt werden, um so gewünschte Druckeigenspannungen an der Oberfläche zu erzielen. Demnach wird ein Verfahren zum Verfestigungsstrahlen von Metallbauteilen, insbesondere von einem Kraftfahrzeugstabilisator vorgeschlagen, welches durch die Verfahrensschritte
- Bereitstellen eines Metallbauteils und Einlegen des Metallbauteils in eine Strahlanlage, wobei das Metallbauteil vor dem Strahlvorgang erwärmt wird,
- Vorstrahlen mit einer definierbaren Vorstrahlgeschwindigkeit und
- anschließendes Verfestigungsstrahlen mit einer definierbaren Verfestigungsstrahlgeschwindigkeit
geprägt ist, wobei die Verfestigungsstrahlgeschwindigkeit größer ist als die Vorstrahlgeschwindigkeit. Da das Strahlen mittels der Geschwindigkeit gesteuert wird, ist die Dimension der Verfestigung nicht immer definierbar.

Zu diesem Verfahren ist aus fachmännisch Sicht ergänzbar, dass nach der bereits oben zitierten DE 102012100279 A1 mittels Vorschubhärten ungleichmäßige Härtezonen vermieden und ungleichmäßige Spannungen innerhalb des zu härtenden Werkstücks klein gehalten werden. Die Anpassung der Querschnittsform eines Induktors an die Querschnittsform der zu härtenden Zahnstange erzeugt Härtezonen, die zum ungehärteten Bereich eine ebene Grenzfläche aufweisen. Diese ebene Grenzfläche soll eine gleichmäßige Spannungsverteilung im Werkstück bewirken, wodurch die Zahnstange eine hohe mechanische Stabilität erhalten kann, jedoch dies mit relativ hohen Kosten verbunden ist.

Es würde auch eine beide Verfahren umfassende Aggregation damit das Problem des in der Verzahnung vorliegenden "chaotischen" Eigenspannungsverlaufs mit Spannungsspitzen allein unzureichend oder gar nicht lösen können.

Andererseits wird nach DE 10140444 A1 ein nachträgliches Verfestigungsstrahlen zum Erhöhen der dynamischen Belastbarkeit eines verzahnten Bauteils abgelehnt, um die Zahnflankentragfähigkeit im Bereich des Zahnfußes erhöhen zu können.

Nach Auswertung dieser gegenüber dem hier zu lösenden Problem andere Wege und Ziele verfolgende Maßnahmen, die bestenfalls Einzellösungen darstellen, erhält der Fachmann keine Anregungen, um die bisher in der Praxis bei der Herstellung von Lenkungszahnstangen angesprochene "chaotische" Spannungsverteilung zielgerichtet oder definierbar zu beherrschen. Es wird ihm sogar Unsicherheit vermittelt, da das Verfestigungsstrahlen mit einer Strahlbehandlung erzielbare Ergebnis das Werkstück auch nachteilig beeinflussen kann, insbesondere wenn es nicht kontrollierbar ist.

Auch weitere Ausblicke auf Verfahren anderer Fachgebiete, wie z.B. in der DE 19500078 A1 beschrieben, bieten keine Lösungsansätze. Jenes Verfahren betrifft die Verringerung des relativen Bruchmomentes im Bereich von Sollbruchstellen von Bauteilen mittels Kaltverformung durch Festigkeitsstrahlen. Zwar geht man dort davon aus, dass biege- oder torsionsbeanspruchte Bauteile in Querschnittsübergängen Spannungsspitzen aufweisen, die ohne entsprechende Gegenmaßnahmen auf der Werkstoffseite zum Bruch führen können, jedoch wäre diese Lösung auf hiesige Zahnstangen nicht übertragbar.

Der Fachmann erkennt, dass komplexere Betrachtungen erforderlich sind, weil zulässige oder geforderte Spannungsverteilungen im Rahmen üblicher Arbeitsabläufe technologisch nicht beherrschbar sind. Dazu bedarf es schöpferischer Überlegungen, um das bisher lediglich als Nachbehandlung vorgesehene Verfestigungsstrahlen in die Arbeitsabläufe der Herstellung von Zahnstangen erfolgreich integrieren zu können oder dieses mit den wie oben analysierten oder anderen Maßnahmen zu kombinieren. Denn die Analyse des Standes der Technik zeigt auch, dass die in der Praxis bei der Herstellung von Lenkungszahnstangen gewonnenen Erfahrungen nicht durch rein rechnerisch bestimmte Technologien ersetzbar sind. Weiterhin ist ein progressives Maß von "trial and error" erforderlich.

Es wurde zwar in einer frühen Quelle von Neuber (Forschung im Ingenieurwesen, Bd. 29 (1963)) über die Spannungsverteilung in Zahnstangen gelehrt, dass mit Hilfe komplexer Rechenverfahren exakte Spannungsfunktionen gewonnen werden können, die für die Spannungsverteilung in Zahnstangen mit beliebigem Profil und bei beliebigem Kraftangriff zu neuen Berechnungsgrundlagen führen können. Jedoch findet der nach neuen Berechnungsgrundlagen suchende Fachmann für die Praxis keine dementsprechenden Lösungsansätze.

Aus der JP 2013241961 A wird eine Bearbeitung von Zähnen eines Getrieberades vorgeschlagen. Sie zielt bei Getriebezahnrädern darauf ab, positiv auf die Verschleißfestigkeit der ZahnOberflächen und Reduzierung der Rauigkeit einzuwirken. Deshalb sollen dort die Oberflächen bei anliegenden Spannungen von >800 MPa mittels Shot Peening behandelt und die Druckspannungen u.a. mittels Röntgenstrahlung gemessen werden.

Aus der Zusammenschau der oben untersuchten Lösungen zum Stand der Technik lässt sich keine Lösung kombinieren, die die nach dem Härten und Richten einer Zahnstange in der Verzahnung vorliegenden nachteiligen, undefinierbaren oder ungleichen wie "chaotischen" Spannungszustände mit insbesondere nachteiligen Zugspannungen und deren Spannungsspitzen vermeidet. Somit werden erforderliche Optimierungen des Masse- und Materialeinsatzes bei Vermeidung späterer Risse oder Brüche einer Zahnstange nicht ohne Weiteres realisierbar. Demnach ist eine neue Lösung zu finden, die
o den Anforderungen der Gewichtseinsparung bei Zahnstangen durch Minimierung der Zahnstangendurchmesser und damit auch des Hüllkreisdurchmessers der Verzahnung bei weitgehend gleichmäßiger Spannungsverteilung und ohne Gefügeveränderung gerecht wird,
o den Nachteil einer sinkenden Dauerfestigkeit der Zahnstange und der Bildung von Rissen oder Brüchen vermeidet und
o ein Potenzial zur Durchmesserminimierung schafft.

### Darstellung der Erfindung

Der Erfindung liegt die Aufgabe zugrunde, das nach dem Härten und/oder Richten der Zahnstange vorliegende Spannungssystem mit chaotischem Eigenspannungsverlauf von Zug- und Druckspannungen durch für sich zum Teil bekannte Technologien und Arbeitsgänge in einer verschmelzenden Schrittfolge eines Bearbeitungszuges in ein die Festigkeit und den Materialeinsatz wie den Durchmesser der Zahnstange optimierendes Spannungssystem umzuwandeln, und zwar ohne Veränderung oder Zerstörung der Gefügebestandteile des jeweiligen Stahls.

Dabei sollen
- röntgenographische Untersuchungen, wie allgemein bekannt und z.B. gemäß US 2006/0048867 A1 speziell angewandt, und/oder
- das für sich bekannte und bisher als Nachbehandlung gedachte oder nach einer technologischen Wartezeit zur Vermeidung von Rissbildungen angewendete Strahlen (Shot Peening) wie Kugelstrahlen oder Verfestigungsstrahlen von Metallbauteilen (z.B. wie oben nach DE 102013218413 A1, DE 102011 055104 B4, DE 10140444 A1) zur Einbringung von Druckspannungen
sowohl in einen kontinuierlichen Ablauf der Arbeitsgänge eingebunden als auch mit einem im Werkstück erzeugenden funktional verschmelzenden neuen Effekt einer gleichmäßigeren Spannungsverteilung gezielt verwendet werden.

Erfindungsgemäss wird die Aufgabe durch das Verfahren nach dem Anspruch 1 und den abhängigen Ansprüchen 2 bis 13 gelöst.

Das erfinderische Prinzip des Verfahrens zur Bearbeitung einer aus einem metallischen Werkstoff hergestellten Zahnstange, bei der nach dem Härten oder dem Härten und Richten mindestens im Bereich einer Verzahnung zunächst ein "chaotischer" Eigenspannungsverlauf von Zugspannungen und Druckspannungen anliegt, besteht darin, dass ohne Gefügeveränderung in einer funktional verschmelzenden Schrittfolge eines Bearbeitungszuges besagter Eigenspannungsverlauf in einen zulässigen Eigenspannungsverlauf umgewandelt wird und dabei mindestens der Bereich der Verzahnung eine Vorverspannung mit definiert eingebrachten Druckeigenspannungen ohne Zugspannungen erhält, so dass ein physikalisches System mit Spannungswerten von Druckeigenspannungen und dem zulässigen Eigenspannungsverlauf einer weitgehend einheitlichen Spannungsverteilung oder Spannungsebene im Bereich der Verzahnung vorliegt. Eingehend auf die oben ausgewertete AT 515352 A1 (DE 102014225995 A1), wonach dort zwar für ein Sinterbauteil mit einer Verzahnung ein maximaler Wert von Druckeigenspannungen zur Verbesserung der Dauerfestigkeit des Sinterbauteils möglich wird, wird hier erfindungsgemäß die Beherrschung von Spannungszuständen ohne Gefügeveränderung des Stahls erreicht, so dass die hiesige Technologie sich davon schon prinzipiell und qualitativ unterscheidet.

Dafür werden nach dem Härten oder dem Härten und Richten in mindestens einer Zahnstange in ausgewählten Abschnitten der Verzahnung in einer Axialrichtung angefallene Zugspannungen und in einer Querrichtung angefallene Druckspannungen in gemessenen Werten von Axialspannungen und Querspannungen zur Definition des "chaotischen" Spannungsverlaufs als Referenz- oder theoretisch anzunehmender Spannungsverlauf für parametergleich und erfindungsgemäß zu bearbeitende Lose oder Serien von Zahnstangen verwendet, und zwar im Rahmen einer technologischen Vorbereitung der jeweiligen Lose oder Serien.

Mit dem Verfahren wird berücksichtigt, dass eine gattungsgemäße Zahnstange regelmäßig nach den prinzipiellen Arbeitsgängen, wie Kurzstück ablängen, Verzahnung in einem Abschnitt anbringen, Härten, Richten und vor der Endenbearbeitung der Stirnseiten, dem Schleifen, Finishing, der Rissprüfung und dem Waschen das hier "chaotisch" bezeichnete Spannungssystem aufweist. Der Fachmann hat die in einer Zahnstange nach Anbringung der Verzahnung und das Härten herrschenden Zug- und Druckspannungen erkannt, um Sie hier der Ausdehnung nach ermitteln zu können, und zwar zerstörungsfrei durch röntgenografische Eigenspannungsmessung. Besagter Eigenspannungsverlauf wird durch röntgenographische Messungen in mindestens einer Randschicht der Abschnitte der Verzahnung in deren Axialrichtung und Querrichtung in einer Tiefe von < 100 µm gemessen.

Da die röntgenographische Spannungsmessung nach dem Prinzip der Ermittlung der Gitterdehnungen als Folge von Restspannungen in einem kristallinen Gitter erfolgt, ist eine Messung als Funktion in einem Koordinatensystem durchzuführen, wonach die Spannungen aus einem mehrachsigen Spannungszustand ermittelt werden.

Der verzahnte Bereich erfordert eine besondere Einstellung des Röntgen-Gerätes wie z.B. eines Diffraktometers auf das Werkstück und den Werkstoff, umfassend solche Kriterien, wie
o die Auswahl der zu bestrahlenden Flächen und
o die Bestimmung der Messungen betreffend die Messpositionen, wie Interferenzlinien, Winkelbereiche, Kippungen.

Demzufolge werden für die röntgenographische Messungen der Eigenspannungen die ausgewählten (Randschicht-)Abschnitte der Verzahnung bestimmt, um dort in deren Axialrichtung und Querrichtung die vorliegenden Druck- und Zugspannungen in gemessenen Werten von Axialspannungen und Querspannungen erfassen zu können.

Zwar sind röntgenographische Messungen in Werkstücken wie oben nach US 2006/0048867 A1 allgemein bekannt, jedoch ist hervorzuheben, dass bei den hier vorliegenden Zahnstangen schon mit dem ersten Bearbeitungsschritt für folgende über das normale fachmännische Handeln hinausgehende Maßnahmen zu sorgen ist, um letztlich die angestrebte, weitgehend gleichmäßige Spannungsverteilung ohne Gefügeveränderung mit verwertbaren Ergebnissen zu erreichen. Die erfindungsgemäß angestrebte gleichmäßige Spannungsverteilung wird nicht nur ohne Gefügeveränderung, sondern auch ohne zusätzliche Mittel (wie z.B. zusätzliche Gasstrahlbehandung nach US 2006/0048867 A) erreicht. Somit sind die erfindungsgemäß besonders strukturierten röntgenographischen Messungen der Eigenspannungen in einen mit dem technologischen Ablauf verschmelzenden Effekt integriert.

Da die in dem vorliegenden "chaotischen" Spannungssystem angefallenen Druck- und Zugspannungen nun messbar und erfassbar sind, können Spannungsspitzen der Zugspannungen theoretisch bestimmt oder angenommen werden, was in den Bearbeitungszug funktional integrierbar ist. Andererseits ist es technologisch rationell, da von einem einzigen "Musterstück" ausgegangen werden kann, d.h. ohne eine Messung des "chaotischen" Spannungssystems an jeder einzelnen Zahnstange der Lose oder Serien vornehmen zu müssen. Von den derart beispielsweise im Bereich von ± <2,0 MPax10³ gemessenen Zugspannungen und Druckspannungen können jeweils mindestens ± 0,5 MPa ×10³ als Spannungsspitzen oder Amplituden definiert werden.

Diese erfindungsgemäße Maßnahme eines ersten technologischen Schrittes charakterisiert schon den besonderen, über den eingangs ermittelten Stand der Technik hinausgehenden erfinderischen Aspekt innerhalb des funktional darauf aufbauenden Bearbeitungszugs, nämlich der Hinblick auf die in der Verzahnung anzustrebende gleichmäßige Spannungsverteilung ohne Gefügeveränderung.

Demnach werden besagte Spannungsspitzen von Zugspannungen durch Einbringung oberflächennaher Druckeigenspannungen gezielt mittels beispielsweise Spannungsspitzenstrahlen wie Glaskugelstrahlen eliminiert, geglättet oder verringert, welches kurzzeitig mit <10s durchführbar ist.

Alternativ können die Spannungsspitzen von Zugspannungen durch induktives Erwärmen wie Stressrelieving eliminiert, geglättet oder verringert werden, wobei das Erwärmen im Bereich von 120...160° C erfolgen kann und vor dem Erwärmen und nach dem Erwärmen Werte gemäß den jeweiligen Spannungszuständen erfassbar sind.

Das induktive Entspannen kann in Abhängigkeit von der Art der Verzahnung erfolgen, wobei eine gleichmäßige oder variable Verzahnung oder die Charakteristik des Werkstoffs berücksichtigt werden können.

Nach dem Spannungsspitzenstrahlen oder induktiven Erwärmen und Eliminieren, Glätten oder Verringern der Spannungsspitzen oder Amplituden erhält mittels anschließendem Stahldrahtkornstrahlen (oder Stahlkorn- wie auch Stahlkugelstrahlen) der Bereich der Verzahnung die Vorverspannung mit den definiert eingebrachten Druckeigenspannungen ohne Zugspannungen, so dass der gewünschte oder zulässige Eigenspannungsverlauf erreicht wird.

Dafür werden die gezielt vorgegebenen Werte von Druckeigenspannungen mittels Strahldruck in Bereichen von ± p1 eingebracht.

Eine erfindungsgemäße Besonderheit der Anwendung dieses Stahldrahtkornstrahlens besteht darin, dass die Druckspannungen in Abhängigkeit der zuvor gemessenen Werte gezielt definiert und im funktionellen Zusammenhang mit dem vorherigen Eliminieren, Glätten oder Verringern der Spannungsspitzen oder Amplituden eingebracht werden. Dabei kann die Verformung, welche der Strahlprozess hervorruft, zugleich in einer definierten Probe bestimmt und kontrolliert werden, indem die Druckspannungen als eine gemessene Intensität mittels der Almenintensitätsmessung, hier auch Almentest genannt, erfasst werden. Hierbei können unter anderem Härte und Form des Strahlmittels, Auftreffwinkel, kinetische Energie des Strahlmittels und eventuelle Behinderungen berücksichtigt werden. Damit wird besagter Almentest zum technologisch integrierten Bestandteil der funktional verschmelzenden Schrittfolge des erfindungsgemäßen Bearbeitungszuges. Somit kann eine einzige Zahnstange die definierte Probe als Almen-Prüfteil für die festgelegte Dauer oder für eine festgelegte Anzahl von Losen oder Serien darstellen.

Durch die wirkungsmäßig innige Verknüpfung und einander unterstützende Kombination dieser Verfahrensschritte im erfindungsgemäßen Bearbeitungszug können die im physikalischen Spannungssystem des Gefüges festgestellten Spannungsspitzen in eine einheitliche Spannungsebene oder Spannungsverteilung mit dem überraschenden Effekt umgewandelt werden, dass in dem besagten Abschnitt der Verzahnung eine Vorverspannung der Verzahnung durch Druckeigenspannungen vorliegt.

Mit der Erfindung können bisher nachteilige Besonderheiten einer variablen Verzahnung gegenüber einer variablen Verzahnung berücksichtigt werden, indem bei variablen Verzahnungen die unterschiedlichen Zahngrundradien und unterschiedlichen Winkel der Zahnflanken durch das erfindungsgemäße Verfahren mit erfasst und abgedeckt werden. Somit kann der ursprüngliche Eigenspannungsverlauf von Zugspannungen und Druckspannungen unter Berücksichtigung der Art der Verzahnung oder auch der Charakteristik eines Werkstoffs der Zahnstange in einen zulässigen Eigenspannungsverlauf umgewandelt werden.

Damit wird die Dimensionierung eines Durchmessers der Zahnstange optimierbar, insbesondere als Lenkzahnstangen von Fahrzeugen zu anderen mit vergleichbaren Achslasten.

Das Verfahren ermöglicht in der Verzahnung Werte von Druckeigenspannungen, die im Bereich von >-0,6 MPa×10³ bis <2,0 MPa ×10³ liegen, so dass schließlich diese Werte ein qualitativ neues Charakteristikum der nach dem Verfahren bearbeiteten Zahnstangen sind.

Die nach dem erfindungsgemäßen Verfahren hergestellte Lenkzahnstange weist demnach im Abschnitt der Verzahnung
a) die in die Verzahnung eingebrachten Druckspannungen,
b) das eine einheitliche Spannungsebene/-verteilung bildende physikalische Spannungssystem und
c) die durch Druckeigenspannungen umgewandelte Vorverspannung der Verzahnung in den Dimensionen
auf, welche Eigenschaften der Lenkzahnstange eine neue, funktional einheitlich verschmolzene Beschaffenheit verleihen. Damit können die gewachsenen Anforderungen der Gewichtseinsparung durch Minimierung des Zahnstangendurchmesser und damit auch des Hüllkreisdurchmessers der Verzahnung erfüllt, trotz Minimierung der Durchmesser die Dauerfestigkeit erhöht und für Automobillenkungen den Forderung nach höheren Achslasten der Fahrzeuge entsprochen sowie die technologischen Kosten niedrig gehalten werden.

### Kurze Beschreibung der Zeichnungen

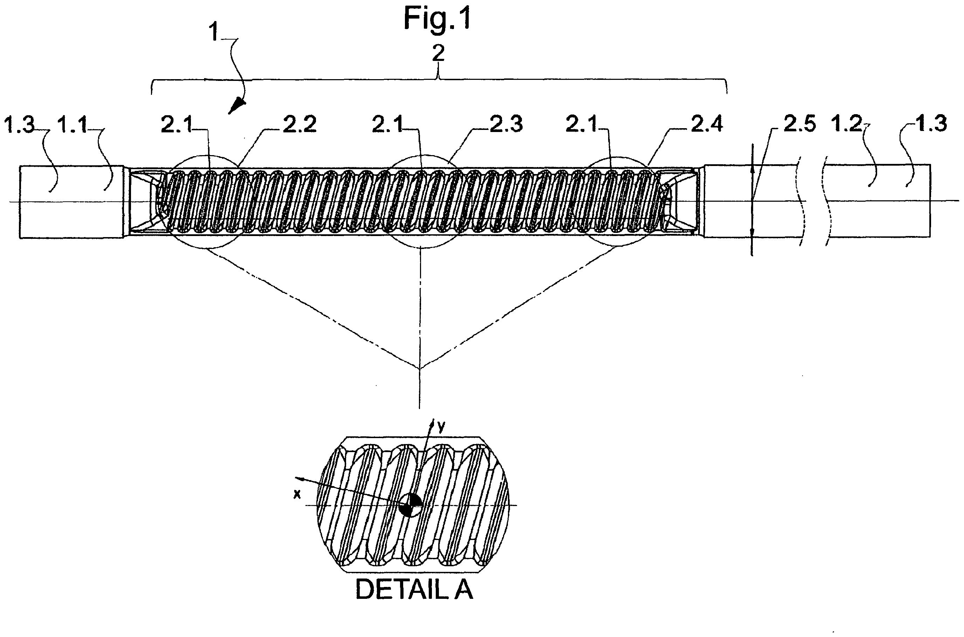

In den Zeichnungen zeigen
- **Fig. 1**: die Draufsicht einer erfindungsgemäß zu bearbeitenden Zahnstange 1, dargestellt als Lenkzahnstange mit einem Messbereich als
- Abschnitt 2.2 der Verzahnung 2, definiert bis zum 5. Zahn ausgehend von einem Zapfen 1.1,
- Abschnitt 2.3 der Verzahnung 2, definiert als Messbereich Mitte, und
- Abschnitt 2.4 der Verzahnung 2, definiert bis zum 5. Zahn ausgehend von einem Schaft 1.2,
aufgeteilt und einem zur Veranschaulichung herausgezogenem Detail A besagter Abschnitte 2.2, 2.3, 2.3 der Verzahnung 2, in denen die Zugspannungen σ+ in einer Axialrichtung x und Druckspannungen σ- in einer Querrichtung y gemessen werden;

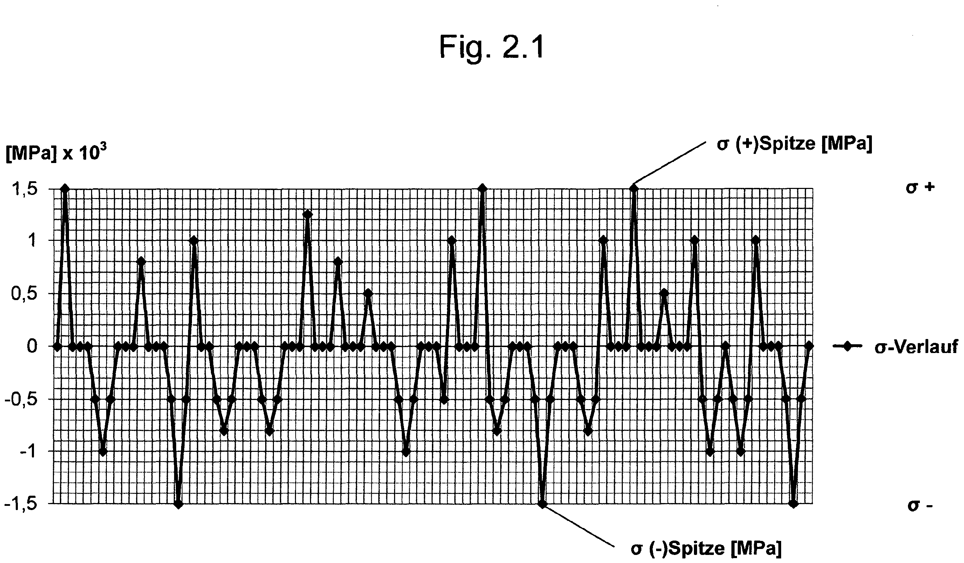

- **Fig. 2.1**: das Diagramm eines gemessenen Spannungsverlaufs σ von Zugspannungen σ+ und Druckspannungen σ- in der Verzahnung 2 gemäß Fig. 1 nach dem Härten und Richten mit Kennzeichnung von Spannungsspitzen σ+_{spitze} der Zugspannungen σ+ und Spannungsspitzen σ-_{spitze} der Druckspannungen σ- der Zahnstange 1 gemäß Fig. 1, welcher als bezeichneter "σ - Verlauf" von einem ausgewählten Musterstück der Zahnstange 1 für zu fertigende Lose, Serien oder Reihen von erfindungsgemäß und parametergleich zu bearbeitenden Zahnstangen 1 als Referenz-Spannungsverlauf σ oder theoretisch anzunehmender Spannungsverlauf σ der parametergleichen Lose oder Serien von Zahnstangen 1 zu Grunde gelegt wird;

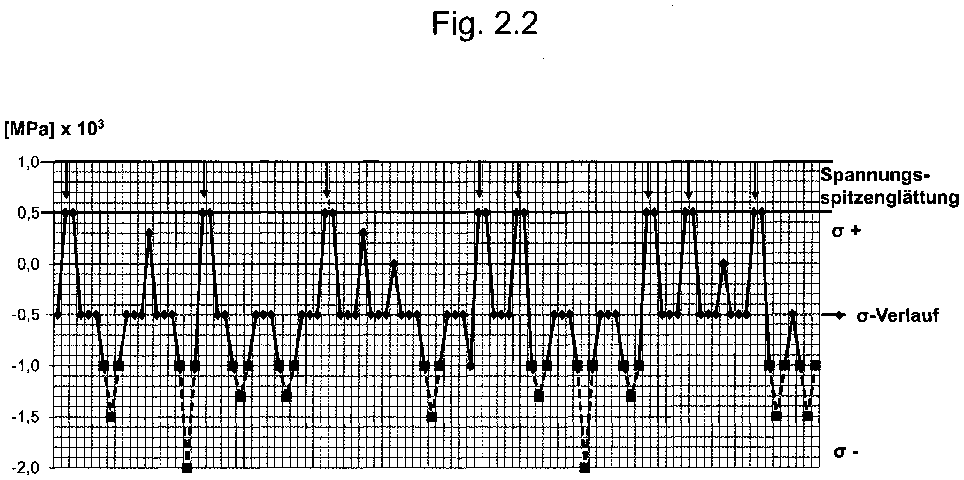

- **Fig. 2.2**: das Diagramm "σ - Verlauf" von Zugspannungen σ+ und Druckspannungen σ- in der Verzahnung 2 der Zahnstange 1 gemäß Fig. 1 nach erfindungsgemäßer Eliminierung oder Glättung von Spannungsspitzen σ+_{spitze} der Zugspannungen σ+, hier mit "Spannungsspitzenglättung" bezeichnet;

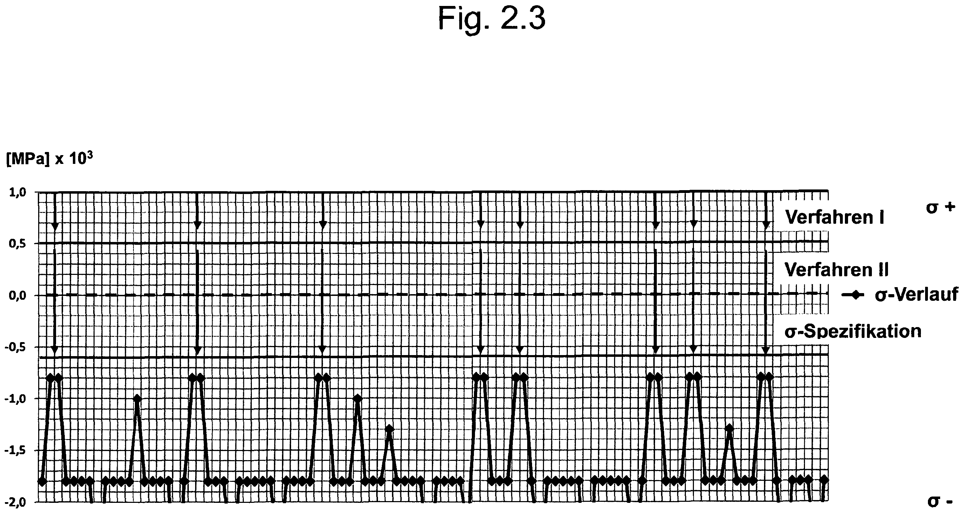

- **Fig. 2.3**: das Diagramm eines zulässigen oder vorgegebenen Spannungsverlaufs σ_{zul} von Druckspannungen σ- in der Verzahnung 2 der Zahnstange 1 gemäß Fig. 1 nach erfindungsgemäßem Bearbeitungszug, wonach der gemäß Fig. 2.1 vorliegende "σ - Verlauf" in einen zulässigen, nicht bezeichneten Spannungsverlauf σ_{zul} umgewandelt wurde und der Bereich der Verzahnung 2 eine Vorverspannung mit definiert eingebrachten, nicht bezeichneten Druckeigenspannungen σ -_{E} ohne Zugspannungen σ + erhalten hat, hier symbolisch mit Pfeilen dargestellt, wonach gemäß
▪ einer ersten, mit "Verfahren I" bezeichneten Stufe die funktional verschmelzende Schrittfolge eines Bearbeitungszuges die hier nicht bezeichneten Spannungsspitzen σ+_{spitze} von Zugspannungen σ + nach Fig. 1 durch Einbringung oberflächennaher, nicht bezeichneter Druckeigenspannungen σ-_{E} gezielt mittels Spannungsspitzenstrahlen geglättet, verringert oder eliminiert werden und
▪ einer zweiten, mit "Verfahren II" bezeichneten Stufe und als Abschluss innerhalb der funktional verschmelzenden Schrittfolge des erfindungsgemäßen Bearbeitungszuges nach dem Spannungsspitzenstrahlen oder induktiven Erwärmen und Eliminieren, Glätten oder Verringern besagter Spannungsspitzen σ+_{spitze} der zulässige Eigenspannungsverlauf σ_{zul} durch ein anschließendes Stahldrahtkornstrahlen entsprechend einer hier bezeichneten "σ-Spezifikation" erreicht wird;
- **Fig. 3**: drei Säulendiagramme mit den in der Verzahnung 2 gemäß Fig. 1 erzielten Meßwerten von Druckeigenspannungen σ -_{E}, wie sie erfindungsgemäß mittels Spannungsspitzenstrahlen oder Stressrelieving und Stahlkornstrahlen mit unterschiedlichen Strahldrücken in einem Bearbeitungszug eingebracht wurden, und zwar in mit I, II, III bezeichneten Blockdarstellungen;
- **Fig. 4**: eine in Diagrammen dargestellte Übersicht erfindungsgemäß erreichter detaillierter Spannungswerte in den Abschnitten 2.2, 2.3 und 2.4 der Verzahnung 2 der Zahnstange 1 gemäß Fig. 1;
- **Fig. 5**: eine schematische Darstellung der beim Glaskugelstrahlen zu vermeidenden, nachteiligen "Nichterreichung" des Zahngrundes.

Nachstehend wird die Erfindung mit Beispielen zur Ausführung näher erläutert.

### Bester Weg zur Ausführung der Erfindung

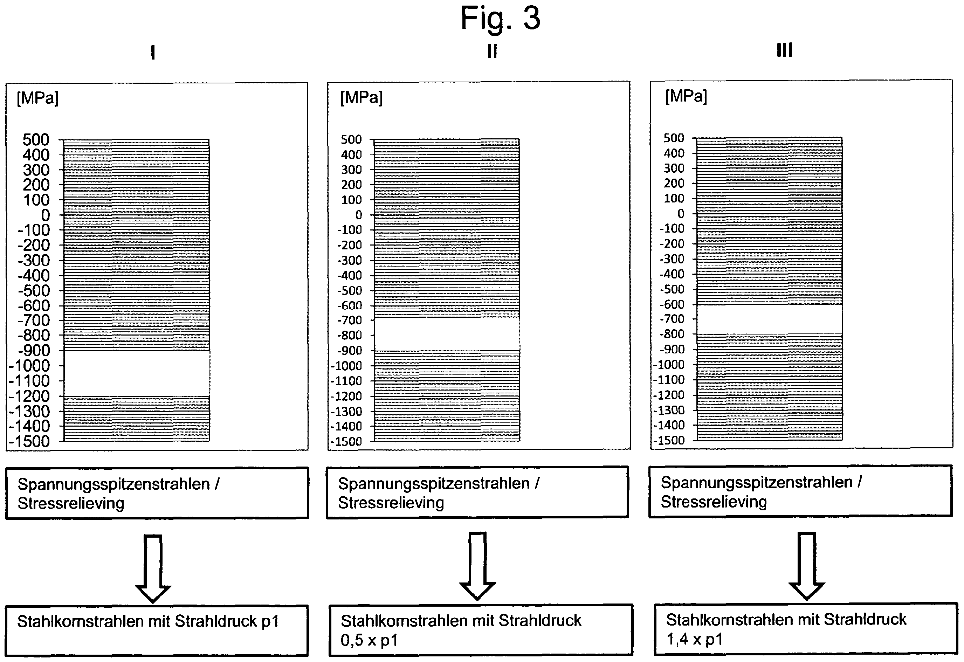

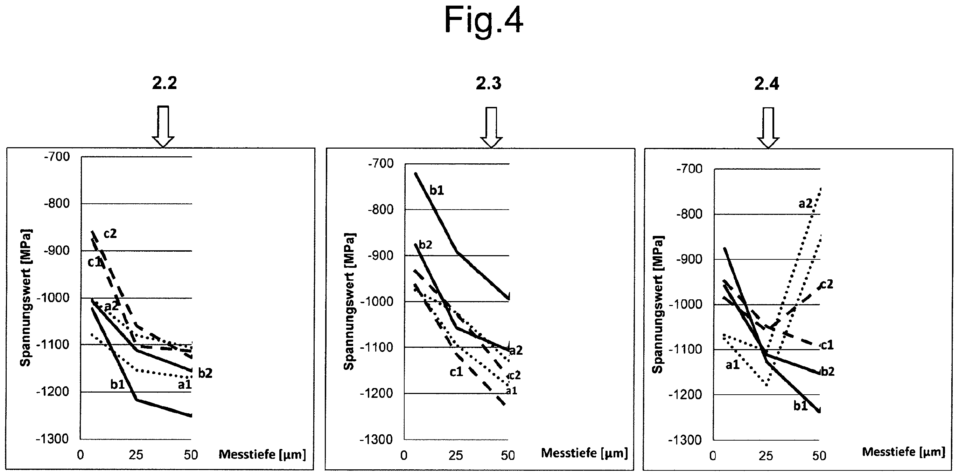

Zum besseren Verständnis der Reproduzierbarkeit einer erfindungsgemäß nach Fig. 1 zu bearbeitenden Zahnstange 1 werden zu den Beispielen technologische Bedingungen nach den Fig. 2.1, 2.2, 2.3, Ergebnisse gemäß Fig. 3 sowie Daten wie ggf. Pflichtendaten nach Fig. 4 dargestellt.

In Fig. 1 wird eine Zahnstange 1 mit zwischen Zapfen 1.1 und Schaft 1.2 durch spanlose Formgebung eingebrachter Verzahnung 2 mit Zähnen 1, durch Stirnseiten 1.3 begrenzter Länge und einem Durchmesser 2.5 zu Grunde gelegt, die nach dem Härten und Richten erfindungsgemäß bearbeitet werden soll.

In Anbetracht der eingangs analysierten Problematik, dass
o nach dem Härten und Richten der Zahnstange 1 in der Verzahnung 2 nachteilige, undefinierbare oder ungleiche hier "chaotisch" bezeichnete Spannungszustände mit einem in Fig. 2.1 dargestellten Spannungsverlauf σ von Zugspannungen σ+ und Druckspannungen σ- in der Verzahnung 2 mit insbesondere nachteiligen Zugspannungen σ+ und deren Spannungsspitzen σ-Spitze vorliegen und
o extern vorgegebene Pflichtendaten, Optimierungen des Masse- und Materialeinsatzes bei Vermeidung späterer Risse oder Brüche der Zahnstange 1 erforderlich werden,
ist die Zahnstange 1 so zu bearbeiten, dass sie
o den Anforderungen der Gewichtseinsparung durch Minimierung der Zahnstangendurchmesser und damit auch des Hüllkreisdurchmessers der Verzahnung bei weitgehend gleichmäßiger Spannungsverteilung und ohne Gefügeveränderung gerecht wird,
o den Nachteil einer sinkenden Dauerfestigkeit der Zahnstange und der Bildung von Rissen oder Brüchen vermeidet und
o überhaupt ein Potenzial zur Durchmesserminimierung bietet.

Durchgängig werden mit den Symbolen
■ σ+ eine Zugspannung,
■ σ- eine Druckspannung,
■ σ-_{E} eine Druckeigenspannung,
■ σ ein Spannungsverlauf, auch "chaotischer" als (Eigen-)Spannungsverlauf und
■ σ_{zul} ein zulässiger, gewünschter, vorgegebener oder erreichter (Eigen-)Spannungsverlauf und bezeichnet.

Erfindungsgemäß ist ohne Gefügeveränderung in einer funktional verschmelzenden Schrittfolge eines Bearbeitungszuges dieser für die Erfüllung der geforderten Parameter nachteilig wirkende (Eigen-)Spannungsverlauf σ in einen zulässigen (Eigen-)Spannungsverlauf σ_{zul} umzuwandeln, wobei (mindestens) der Bereich der Verzahnung 2 eine Vorverspannung mit definiert eingebrachten Druckeigenspannungen σ -_{E} ohne Zugspannungen σ + erhält, so dass im Ergebnis der Bearbeitung ein physikalisches System mit Spannungswerten von Druckeigenspannungen σ-_{E} und dem zulässigen (Eigen-)Spannungsverlauf σ_{zul} einer weitgehend einheitlichen Spannungsverteilung oder Spannungsebene im Bereich der Verzahnung 2 vorliegt.

Dem erfindungsgemäßen Bearbeitungsverfahren ist dabei zugrunde gelegt, dass in der Verzahnung 2 mit Zähnen 1 obligatorisch, d.h. gemessen oder nicht gemessen oder festgestellt oder nicht festgestellt, ein "chaotischer" (Eigen-)Spannungsverlauf σ von Zugspannungen σ+ und Druckspannungen σ- gemäß Fig. 2.1 anliegt. Dieser Zustand ist im Zusammenhang mit der Erfindung an einem Musterstück der Zahnstange 1 wie folgt zu untersuchen und zu definieren. Erfindungsbedeutsam und als quasi technologische Vorbereitung der mit diesen integrierten Schritten zu bearbeitenden Zahnstange 1 gemäß Fig.1 wird der Abschnitt der Verzahnung 2 mit den Zähnen 2.1 (= Länge der Verzahnung 2) in je einen
■ Abschnitt 2.2 als Messbereich bis zum 5. Zahn 2.1 ausgehend vom Zapfen 1.1,
■ Abschnitt 2.3 als Messbereich Mitte und
■ Abschnitt 2.4 als Messbereich bis zum 5. Zahn ausgehend vom Schaft 1.2 aufgeteilt.

Das herausgezogene Detail A der Verzahnung 2 zeigt dazu symbolische Vektorpfeile
■ für die Messung der Zugspannungen σ+ in einer Axialrichtung x und
■ für die Messung der Druckspannungen σ- in einer Querrichtung y.

Auf der Grundlage dieses für die erfindungsgemäße Bearbeitung vorbereitete technologische System werden durch röntgenographische Messungen in den ausgewählten (Randschicht-) Bereichen in Axialrichtung x die Zugspannungen σ+ und in Querrichtung y die Druckspannungen σ- der Verzahnung 2 festgestellt und als gemessene Werte erfasst.

Es sind zwar röntgenographische Messungen in Werkstücken allgemein bekannt, jedoch ist hervorzuheben, dass bei der hier vorliegenden Zahnstange 1 schon für den ersten technologischen Ansatz über das normale fachmännische Handeln hinausgehende Maßnahmen zu entwickeln und zu erproben waren, um letztlich für eine weitgehend gleichmäßige Spannungsverteilung und ohne Gefügeveränderung verwertbare Ergebnisse zu erhalten. Da die röntgenographische Spannungsmessung nach dem Prinzip der Ermittlung der Gitterdehnungen als Folge von Restspannungen in einem kristallinen Gitter erfolgt, konnte hierfür eine Messung in dem hier angegebenen Koordinatensystem erfolgen, wonach die Spannungen aus dem mehrachsigen Spannungszustand ermittelt werden.

Da die Verzahnung 2 eine besondere Einstellung des Röntgen-Gerätes wie z.B. eines Diffraktometers auf die Zahnstange 1 erfordert, sind deshalb solche Kriterien, wie
o die Auswahl der zu bestrahlenden Flächen und
o die Bestimmung der Messungen betreffend die Messpositionen, wie Interferenzlinien, Winkelbereiche, Kippungen
zu beachten.

Demzufolge sind für diesen technologisch vorzubereitenden Schritt die für die röntgenographische Messungen der Eigenspannungen auszuwählenden (Randschicht-) Abschnitte 2.2, 2.3, 2.4 der Verzahnung 2 zu bestimmen. Dort wird in deren Axialrichtung x und Querrichtung y der vorliegende Spannungsverlauf σ von Zugspannungen σ+ und Druckspannungen σ- in der Verzahnung 2 nach dem Härten und Richten mit Kennzeichnung von Spannungsspitzen σ+_{spitze} der Zugspannungen σ+ und Spannungsspitzen σ-_{spitze} der Druckspannungen σ- in gemessenen Werten erfasst.

Dieses erfindungsgemäße Herangehen zeitigt den Vorteil, dass von einem ausgewählten Musterstück der Zahnstange 1 der "chaotische" Spannungsverlauf σ für die dann zu bearbeitenden Lose oder Serien von parametergleich zu bearbeitenden Zahnstangen 1 ein definierter Referenz-Spannungsverlauf σ oder theoretisch anzunehmender Spannungsverlauf σ für die parametergleichen Lose oder Serien von Zahnstangen 1 praxisnah zu Grunde liegt.

In Fig. 2.1 ist ein daraus entwickelter Referenz-Spannungsverlauf σ oder theoretisch anzunehmender Spannungsverlauf σ für parametergleiche Lose oder Serien von Zahnstangen 1 als Diagramm dargestellt, woraus der Spannungsverlauf σ und Werte in der Dimension [MPa] x 10³ von Zugspannungen σ+ und Druckspannungen σ- in der Verzahnung 2 nach dem Härten und Richten mit gekennzeichneten Spannungsspitzen σ+_{spitze} der Zugspannungen σ+ und Spannungsspitzen σ-_{spitze} der Druckspannungen σ- der zum Vergleich heranzuziehenden Zahnstange 1 gemäß Fig. 1 entnehmbar sind. Dieses von einem ausgewählten Musterstück der Zahnstange 1 abgenommene Spannungssystem gilt dann für parametergleich und erfindungsgemäß zu fertigende Lose oder Serien von Zahnstangen 1 als Referenz- oder theoretisch anzunehmender Spannungsverlauf σ mit den Werten in der Dimension [MPa] × 10³ von Zugspannungen σ+ und Druckspannungen σ-.

Verfahrensgemäß ist aus Fig. 2.2 schematisch ersichtlich, dass und wie in der funktional verschmelzenden Schrittfolge eines Bearbeitungszuges Spannungsspitzen oder Amplituden σ+_{spitze} der vorliegenden Zugspannungen σ+ vorliegen: Demnach sind die Spannungsspitzen oder Amplituden σ+_{spitze} von Zugspannungen σ+ in Werten der Dimension [MPa] × 10³ gemäß einer mit Spannungsspitzenglättung bezeichneten ersten Stufe, in Fig. 2.3 mit Verfahren I bezeichnet, definier- und eliminierbar.

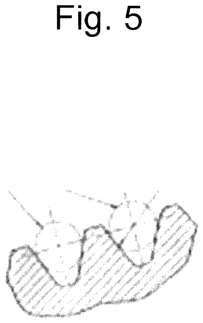

Die funktional verschmelzende Schrittfolge eines Bearbeitungszuges umfasst, dass die Spannungsspitzen σ+_{spitze} von Zugspannungen σ + durch Einbringung oberflächennaher Druckeigenspannungen σ-_{E} gezielt mittels Spannungsspitzenstrahlen wie Glaskugelstrahlen geglättet, verringert oder eliminiert werden, und zwar wie es symbolisch mit Pfeilen gemäß der ersten Stufe so auch in der noch unten näher abzuhandelnden Fig. 2.3 dargestellt ist. Das Spannungsspitzenstrahlen kann kurzzeitig mit <10s durchgeführt werden. Bei dem Glaskugelstrahlen sind die Kugeln des Strahlgutes so zu bemessen, dass im Zahngrund der Verzahnung 2 ein jeweiliger Zahnfußradius bestrahlbar wird, um eine nachteilige, in Fig. 5 schematisch dargestellte "Nichterreichung" des Zahngrundes zu vermeiden.

Alternativ können die Spannungsspitzen σ+_{spitze} von Zugspannungen σ + (und ggf. Spannungsspitzen σ-_{spitze} von Druckspannungen σ -) durch induktives Erwärmen wie Stressrelieving eliminiert, geglättet oder verringert werden. Das Erwärmen kann im Bereich von 120... 160° C erfolgen, wobei vor dem Erwärmen und nach dem Erwärmen Spannungswerte erfassbar sind.

Demnach wird nach dem Spannungsspitzenstrahlen oder Stressrelieving gemäß Fig. 2.2 (zunächst als technologische Zwischenstufe des erfindungsgemäßen Verfahrens) ein (Eigen-)Spannungsverlauf σ ohne Spannungsspitzen σ+_{spitze} erreicht.

Erfindungsgemäß ist in dem Bereich der Verzahnung 2 eine Vorverspannung mit definierten Druck(eigen)spannungen σ -_{E} ohne Zugspannungen σ+ einzubringen, so dass vorausschauend ein physikalisches System mit Spannungswerten von Druck(eigen)spannungen σ-_{E} und dem zulässigen (Eigen-)Spannungsverlauf σ_{zul} einer weitgehend einheitlichen Spannungsverteilung oder Spannungsebene im Bereich der Verzahnung 2 vorliegt. Dazu wird nach dem Spannungsspitzenstrahlen oder induktiven Erwärmen und Eliminieren, Glätten oder Verringern der Spannungsspitzen σ+_{spitze} oder Amplituden der zulässige (ggf. nach den Pflichtendaten zu erreichende) Eigenspannungsverlauf σ_{zul} durch ein anschließendes Stahldrahtkornstrahlen mit einem Strahldruck von p1 erreicht, und zwar in einer zweiten Stufe II gemäß Fig. 2.3 und als Abschluss innerhalb der funktional verschmelzenden Schrittfolge des erfindungsgemäßen Bearbeitungszuges. Damit liegt im Bereich der Verzahnung 2 die Vorverspannung mit den definiert eingebrachten Druckeigenspannungen σ -_{E} ohne Zugspannungen σ + vor, wie es in der Fig. 2.3 im Diagramm gemäß einer σ-Spezifikation
dargestellt ist.

Fig. 3 zeigt Werte von gewünschten Druckeigenspannungen σ -_{E} (und damit einen zulässigen oder nach Pflichtendaten zu erreichenden Eigenspannungsverlauf σ_{zul}) für bearbeitete Zahnstangen 1, wie sie durch den erfindungsgemäßen Bearbeitungszug mittels Spannungsspitzenstrahlen oder Stressrelieving und Stahldrahtkornstrahlen bei Strahldrücken von beispielsweise p1 gemäß Blockdarstellung I, 0,5 × p1 gemäß Blockdarstellung II oder 1,4 × p1 gemäß Blockdarstellung III gezielt eingebracht wurden.

Das hier als Festigkeitsstrahlen funktionierende Stahldrahtstrahlen ist hinsichtlich der gezielt einzubringenden Werte von Druckeigenspannungen σ-_{E} nach dem in den Bearbeitungszug integrierten "Almentest" (ggf. mit vorgeschaltetem Lacktest) zur Sicherung der Qualität beherrschbar. Während des Strahlbetriebes wird der Betriebsdruck kontrolliert, und nach dem "Almentest" liegt eine Zahnstange 1 als quasi Prüfkörper vor, an welchem (siehe Fig. 4) nachweisbar ist, dass im Ergebnis der erfindungsgemäß verknüpften Schritte mit den Werten a1 = Spannung axial Anfang, a2 = Spannung quer Anfang, b1 = Spannungt axial Mitte, b2 = Spannung quer Mitte, c1 = Spannung axial Ende, c2 = Spannung quer Ende eine zulässige Spannungsebene oder Spannungsverteilung in den Abschnitten 2.1, 2.2, 2.3 der Verzahnung 2 besteht.

Da nun in den Abschnitten 2.2, 2.3, 2.4 der Verzahnung 2 Werte von Druckeigenspannungen σ-_{E} im Bereich von beispielsweise >-0,6 MPa×10³ bis <-2,0 MPa ×10³ möglich sind, gestattet die Erfindung im Hinblick auf die konstruktive Entwicklung von Zahnstangen 1 das Folgende:
Bei der herkömmlichen Bearbeitung einer Zahnstange 1 unterzog der konstruierende Fachmann deren Querschnittsbetrachtung und Optimierung regelmäßig nach der Zahnstangenbeanspruchung, einer Biegelinie für den vorgegebenen Lastfall und den Dauerschwingversuchen, um das entsprechende Widerstandsmoment aufzubringen und die gewünschte Dauerschwingfestigkeit oder Lebensdauer zu erzielen. So ermittelte er beispielsweise einen Zahnstangendurchmesser D von 28 mm im Bereich der Verzahnung 2₋ Dabei konnten zwar die Druckeigenspannungen -σ -_{E} oder Höhe und Richtung oberflächennaher Spannungen σ unberücksichtigt bleiben, jedoch blieben Zugspannungen σ+ in der Verzahnung 2, also der negative "chaotische" Spannungsverlauf σ von Zugspannungen σ+ und Druckspannungen σ-(siehe Fig. 2.1) nachteilig erhalten.

Der nun die erfindungsgemäße technologische Lehre aufnehmende Fachmann kann die konstruktive Optimierung von Zahnstangen 1 vorteilhaft vertiefen, wobei er
o ausschlaggebende Faktoren für Dauerschwingfestigkeit und Lebensdauer, wie Querschnittsfläche und Widerstandsmoment der Zähne 2.1 am Zahngrund (inkl. Zahnfußradien und deren entsprechende Kerbwirkung) und Verzahnungsbreite, Zahnfußebene bzw. -fläche untersuchen wird,
o des Weiteren den Eigenspannungsverlauf σ von Zugspannungen σ+ und Druckspannungen σ- in Abhängigkeit von einer variablen Verzahnung 2 iᵥₐᵣ oder konstanten Verzahnung 2 iₖₒₙₛₜₐₙₜ oder der Charakteristik eines Werkstoffs der Zahnstange 1 in den zulässigen Eigenspannungsverlauf σ_{zul} umwandeln wird,
o durch das gezielte Einbringen von oberflächennahen Druckeigenspannungen σ-_{E} (z.B. 0,02 mm unter der Oberfläche) die Zugspannungen σ+ eliminiert und den Durchmesser D der Zahnstangen 1 auf beispielsweise 26 mm minimiert und
o sogar die Dimensionierung des Durchmessers D der Zahnstange 1 wie Lenkzahnstange eines Fahrzeuges bei gleichen Achslasten eines Fahrzeuges optimieren kann.

Das wird möglich, weil die verfahrensgemäße Erfindung eine Zahnstange 1 schafft, die Werte von Druckeigenspannungen σ-_{E} im Bereich von >-0,6 MPax10³ bis <-2,0 MPa ×10³ zulässt.

In Fig. 4 ist dargestellt, wie die im physikalischen Spannungssystem des Gefüges ursprünglich nachteilig wirkenden Spannungsverhältnisse im Ergebnis der erfindungsgemäß verknüpften Schritte mit den oben definierten Werten a1, a2; b1, b2; c1, c2 in eine zulässige Spannungsebene oder Spannungsverteilung in den Abschnitten
o 2.2, gemessen am 5. Zahn ausgehend vom Zapfen 1.1,
o 2. 3, im Messbereich Mitte,
o 2. 4, gemessen am 5. Zahn ausgehend vom Schaft 1.2
der Verzahnung 2 umgewandelt wurden. Damit können auch Vorgabewerte wie externe Pflichtendaten zu den erreichten Ist-Werten verglichen und als erfüllt nachgewiesen werden, dass also in der Verzahnung 2 eine vorteilhafte Vorverspannung durch Druckeigenspannungen-σ -_{E} ohne beeinträchtigende Zugspannungen σ+ anliegt.

### Gewerbliche Anwendbarkeit

Die Erfindung ist technologisch mit relativ niedrigen Kosten anwendbar und ermöglicht, den Anforderungen der Gewichtseinsparung bei Zahnstangen durch Minimierung der Zahnstangendurchmesser nachzukommen. Der Dauerfestigkeit von Zahnstangen wird entsprochen, Risse oder Brüche in der Verzahnung werden vermieden. Insbesondere für Automobillenkungen kann die Forderung nach höheren Achslasten der Fahrzeuge realisiert werden.

### Liste der Bezugszeichen und verwendeten Symbole

- 1: = Zahnstange
- 1.1: = Zapfen
- 1.2: = Schaft
- 1.3: = Stirnseite
- 2: = Verzahnung
- 2.1: = Zahn
- 2.2: = Abschnitt der Verzahnung 2 als Messbereich ausgehend vom Zapfen 1.1
- 2.3: = Abschnitt der Verzahnung als Messbereich Mitte
- 2.4: = Abschnitt der Verzahnung 2 als Messbereich ausgehend vom Schaft 1.2
- 2.5: = Durchmesser der Zahnstange 1
- x: = Axialrichtung
- y: = Querrichtung
- σ: = Spannungsverlauf, auch "chaotischer" (Eigen-)Spannungsverlauf
- σ_{zul}: = zulässiger, gewünschter, vorgegebener (Eigen-)Spannungsverlauf, auch mit "σ-Spezifikation" bezeichnet
- σ+: = Zugspannung
- σ-: = Druckspannung
- σ-_{E}: = Druckeigenspannung
- σ+_{spitze}: = Spannungsspitze oder Amplitude von Zugspannungen
- σ+ₓ: = Axialspannung
- σ+_{y}: = Querspannung
- iᵥₐᵣ: = variable Verzahnung
- iₖₒₙₛₜₐₙₜ: = konstante Verzahnung
- ± p1: = variierbarer Strahldruck
- a1: = Spannungswert axial Anfang
- a2: = Spannungswert quer Anfang
- b1: = Spannungswert axial Mitte
- b2: = Spannungswert quer Mitte
- c1: = Spannungswert axial Ende
- c2: = Spannungswert quer Ende

## Patentansprüche

1. Verfahren zur Bearbeitung einer aus einem metallischen Werkstoff hergestellten Zahnstange (1), bei der nach dem Härten oder dem Härten und Richten mindestens im Bereich einer Verzahnung (2) zunächst ein "chaotischer" (Eigen) Spannungsverlauf (σ) von Zugspannungen (σ+) und Druckspannungen (σ-) anliegt, wobei ohne Gefügeveränderung in einer funktional verschmelzenden Schrittfolge eines Bearbeitungszuges der (Eigen-) Spannungsverlauf (σ) in einen zulässigen (Eigen-)Spannungsverlauf (σzul) umgewandelt wird und mindestens der Bereich der Verzahnung (2) eine Vorverspannung mit definiert eingebrachten Druckeigenspannungen (σ -E) ohne Zugspannungen (σ +) erhält, so dass ein physikalisches System mit Spannungswerten von Druckeigenspannungen (σ-E) und dem zulässigen (Eigen-)Spannungsverlauf (σ zul) einer weitgehend einheitlichen Spannungsverteilung oder Spannungsebene im Bereich der Verzahnung (2) vorliegt, wobei
a) Spannungsspitzen (σ+spitze) oder Amplituden der vorliegenden Zugspannungen (σ+) durch Einbringung oberflächennaher Druckeigenspannungen (σ-E) gezielt mittels Spannungsspitzenstrahlen wie Glaskugelstrahlen oder durch induktives Erwärmen wie Stressrelieving eliminiert, geglättet oder verringert werden und
b) mittels anschließendem Stahldrahtkornstrahlen oder Stahlkorn- wie auch Stahlkugelstrahlen der Bereich der Verzahnung (2) die Vorverspannung mit den definiert eingebrachten Druckeigenspannungen (σ -E) ohne Zugspannungen (σ +) erhält, so dass der zulässige Eigenspannungsverlauf (σzul) erreicht wird.

2. Verfahren nach Anspruch 1, wobei nach dem Härten oder dem Härten und Richten in mindestens einer Zahnstange (1) in auswählbaren Abschnitten (2.2, 2.3, 2.4) der Verzahnung (2) in einer Axialrichtung (x) angefallene Zugspannungen (σ+) und in einer Querrichtung (y) angefallene Druckspannungen (σ-) in gemessenen Werten von Axialspannungen (σ+x) und Querspannungen (σ -y) zur Definition des "chaotischen" Spannungsverlaufs (σ) als Referenz- oder theoretisch anzunehmender Spannungsverlauf (σ) für parametergleich und erfindungsgemäß zu bearbeitende Lose, Serien oder Reihen von Zahnstangen (1) verwendet werden.

3. Verfahren nach Anspruch 1 oder 2, wobei der Eigenspannungsverlauf (σ) durch röntgenographische Messungen in mindestens einer Randschicht der Abschnitte (2.2, 2.3, 2.4) der Verzahnung (2) in deren Axialrichtung (x) und Querrichtung (y) gemessen wird.

4. Verfahren nach einem der Ansprüche 1 bis 3, wobei die Axialspannungen (σ+x) und die Querspannungen (σ-y) bis zu einer definierten Tiefe von <100 µm gemessen werden.

5. Verfahren nach einem der Ansprüche 1 bis 4, wobei nach dem Härten oder dem Härten und Richten in den Abschnitten (2.2, 2.3, 2.4) Zugspannungen (σ+) und Druckspannungen (σ-) im Bereich von ± < 2,0 MPa × 10³ gemessen werden, von denen jeweils mindestens ± 0,5 MPa × 10³ als Spannungsspitzen (a+_{spitze}) oder Amplituden definiert werden.

6. Verfahren nach Anspruch 1, **dadurch gekennzeichnet, dass** das Spannungsspitzenstrahlen kurzzeitig mit <10s durchgeführt wird.

7. Verfahren nach Anspruch 1, **dadurch gekennzeichnet, dass** das Erwärmen im Bereich von 120...160° C erfolgt, wobei vor dem Erwärmen und nach dem Erwärmen Werte gemäß den jeweiligen Spannungszuständen erfasst werden.

8. Verfahren nach Anspruch 1, **dadurch gekennzeichnet, dass** die gezielt vorgegebenen Werte von Druckeigenspannungen (σ-_{E}) mittels Strahldruck in Bereichen von ± p1 eingebracht werden.

9. Verfahren nach einem der Ansprüche 1 bis 8, **dadurch gekennzeichnet, dass** in den Abschnitten (2.2, 2.3, 2.4) Werte von Druckeigenspannungen (σ-_{E}) im Bereich von >-0,6 MPa ×10³ bis <2,0 MPa ×10³ anliegen.

10. Verfahren nach einem der Ansprüche 1 bis 9, **dadurch gekennzeichnet, dass** die definiert eingebrachten Druckeigenspannungen (σ-_{E}) als eine gemessene Intensität mittels der Almenintensitätsmessung kontrolliert und erfasst werden.

11. Verfahren nach einem der Ansprüche 1 bis 10, **dadurch gekennzeichnet, dass** der Spannungsverlauf (σ) gemäß der Almenintensitätsmessung als Referenz- oder theoretisch anzunehmender Spannungsverlauf σ für parametergleich und erfindungsgemäß zu bearbeitende Lose oder Serien von Zahnstangen (1) verwendet wird.

12. Verfahren nach einem der Ansprüche 1 bis 11, **dadurch gekennzeichnet, dass** der Eigenspannungsverlauf (σ) von Zugspannungen (σ+) und Druckspannungen (σ-) in Abhängigkeit von der Art einer Verzahnung (2) iᵥₐᵣ oder iₖₒₙₛₜₐₙₜ oder der Charakteristik eines Werkstoffs der Zahnstange (1) in einen zulässigen Eigenspannungsverlauf (σ_{zul}) umgewandelt wird.

13. Verfahren nach einem der Ansprüche 1 bis 12, **dadurch gekennzeichnet, dass** die Dimensionierung eines Durchmessers (D) der Zahnstange (1) wie Lenkzahnstange eines Fahrzeuges gegenüber gleichen Achslasten eines anderen Fahrzeuges optimiert wird.

## Claims

1. A method for machining a rack (1) made of a metallic material, where in the rack (1) after hardening, or after hardening and straightening, at least in a region of a gear teeth (2) initially a chaotic (inherent) stress profile (σ) of tensile stresses (σ+) and compressive stresses (σ-) is present, wherein without change in microstructure in a functionally combined series of steps of a machining operation the chaotic (inherent) stress profile (σ) is converted into a permissible (inherent)stress profile (azul) and at least the region of the gear teeth (2) receives a pre-stress with defined introduced compressive inherent stresses (σ-E) without the tensile stresses (σ+), so that a physical system with stress values of compressive inherent stresses (σ-E) and the permissible (inherent) stress profile (σzul) of a substantially uniform stress distribution or stress plane is present in the region of the gear teeth (2),
wherein
a) stress peaks (σ+ spitze) or amplitudes of the present tensile stresses (σ+) are intentionally eliminated, smoothed or reduced by introducing near-surface inherent compressive stresses (σ-E) by shot peening like glass-bead beams or by inductive heating such as stress relieving, and
b) by way of subsequent steel wire shot blasting or steel shot blasting as well as steel shot peening, the region of the gear teeth (2) attains the pre-stress with the defined introduced compressive inherent stresses (σ-E) without the tensile stresses (σ+) so that that the permissible inherent stress profile (σzul) is attained.

2. The method of claim 1, wherein after hardening, or after hardening and straightening, in at least one rack (1) in selectable sections (2.2, 2.3, 2.4) of the gear teeth (2), the tensile stresses (σ+) in an axial direction (x) and the compressive stresses (σ-) in a transverse direction (y) in form of measured values of axial stresses (σ+x) and transverse stresses (σ y) are used to define the chaotic stress profile (σ) as a reference stress profile or a theoretically assumed stress profile (σ) for lots, series or sets of the racks (1) to be machined with identical parameters.

3. The method of claim 1 or 2, wherein the inherent stress profile (σ) is measured by X-ray diffraction measurements in at least one near-surface layer of the sections (2.2, 2.3, 2.4) of the gear teeth (2) in the axial direction (x) and the transverse direction (y).

4. The method of any one of claims 1 to 3, wherein the axial stresses (σ+x) and the transverse stresses (σ-y) are measured up to a defined depth of <100 µm.

5. The method of any one of claims 1 to 4, wherein after hardening, or after hardening and straightening, in the sections (2.2, 2.3, 2.4) tensile stresses (σ+) and compressive stresses (σ-) in the range of ± <2.0 MPa × 10³ are measured, of which at least ± 0.5 MPa × 10³ are defined as the stress peaks (σ+_{spitze}) or amplitudes.

6. The method of claim 1, wherein the shot peening is performed briefly for <10s.

7. The method of claim 1, wherein the heating takes place in a range of 120°C to 160°C, wherein, before the heating and after the heating, values according to respective stress states are measured.

8. The method of claim 1, wherein the defined introduced values of compressive inherent stresses (σ-E) are introduced by beam pressure in a range of ± p1.

9. The method of any one of claims 1 to 8, wherein in the sections (2.2, 2.3, 2.4) values of compressive inherent stresses (σ-E) are present in a range of > -0.6 MPa × 10³ to < 2.0 MPa × 10³.

10. The method of any one of claims 1 to 9, wherein the defined introduced compressive inherent stresses (σ-E) are controlled and detected as a measured intensity by way of an Almen intensity measurement.

11. The method of any one of claims 1 to 10, wherein the stress profile (σ) according to the Almen intensity measurement is used as a reference stress profile or a theoretically assumed stress profile (σ) for lots or series of racks (1) to be machined with identical parameters.

12. The method of any one of claims 1 to 11, wherein the inherent stress profile (σ) of tensile stresses (σ+) and compressive stresses (σ-) is converted into a permissible inherent stress profile (azul) depending on the type of gear teeth (2) iᵥₐᵣ or iₖₒₙₛₜₐₙₜ or the characteristic of a material of the rack (1).

13. The method of any one of claims 1 to 12, wherein dimensioning of a diameter (D) of the rack (1) as steering rack of a vehicle is optimized compared to identical axle loads of another vehicle.

## Revendications

1. Procédé, destiné à usiner une crémaillère (1) fabriquée en une matière métallique, lors duquel, après la trempe ou la trempe et le dressage est appliquée au moins dans la zone d'une denture (2) d'abord une courbe de contraintes (σ) (résiduelles) « chaotique » de contraintes en traction (σ+) et de contraintes en compression (σ-), sans aucune modification de la microstructure, dans une succession d'étapes se combinant fonctionnellement d'une phase d'usinage, la courbe de contraintes (σ) (résiduelles) étant transformée en une courbe de contraintes (σzul) (résiduelles) admissibles et au moins la zone de la denture (2) recevant une précontrainte par contraintes résiduelles en compression (σ -E) introduites de manière définie, sans contraintes en traction (σ +), de sorte qu'un système physique de valeurs de contraintes comprenant des contraintes en compression (résiduelles) (σ-E) et la courbe de contraintes (σ zul) (résiduelles) admissibles avec une distribution de contraintes ou un plan de contraintes sensiblement uniforme soit présent dans la zone de la denture (2),
a) par l'introduction de contraintes résiduelles en compression (σ-E) plus proches de la surface, des pics de contrainte (σ+spitze) ou des amplitudes des contraintes en traction (σ+) présentes étant éliminé(e)s, lissées ou réduites de manière ciblée au moyen de sablage de pics de contrainte, comme le grenaillage de verre ou par chauffage par induction, comme le stressrelieving et
b) au moyen du sablage au fil d'acier ou du sablage aux grenailles d'acier ou aux billes d'acier, la zone de la denture (2) recevant la précontrainte avec les contraintes résiduelles en compression (σ -E) introduites de manière définie, sans contraintes en traction (σ +), de sorte que la courbe de contraintes résiduelles (σzul) admissibles soit obtenue.

2. Procédé selon la revendication 1, après la trempe ou la trempe et le dressage, dans au moins une crémaillère (1), dans des parties (2.2, 2.3, 2.4) sélectionnables de la denture (2), des contraintes en traction (σ+) produites dans une direction axiale (x) et des contraintes en compression (σ-) produites dans une direction transversale (y) étant utilisées dans des valeurs mesurées de contraintes axiales (σ+x) et de contraintes transversales (σ -y) pour la définition de la courbe de contraintes (σ) chaotique en tant que courbe de contrainte (σ) de référence ou théoriquement supposable pour les lots, les séries ou les gammes de crémaillères (1) qui devront être usiné(e)s avec des paramètres identiques et selon l'invention.

3. Procédé selon la revendication 1 ou 2, la courbe de contraintes (σ) résiduelles étant mesurée par des mesures radiographiques dans au moins une couche marginale des parties (2.2, 2.3, 2.4) de la denture (2) dans leurs direction axiale (x) et direction transversale (y).

4. Procédé selon l'une quelconque des revendications 1 à 3, les contraintes axiales (σ+x) et les contraintes transversales (σ-y) étant mesurées jusqu'à une profondeur définie de <100 µm.

5. Procédé selon l'une quelconque des revendications 1 à 4, après la trempe ou la trempe et le dressage, dans les parties (2.2, 2.3, 2.4), des contraintes en traction (σ+) et des contraintes en compression (σ-) de l'ordre de ± < 2,0 MPa × 10³ étant mesurées, dont chaque fois au moins ± 0,5 MPa × 10³ sont définies en tant que pics de contrainte (a+_{spitze}) ou amplitudes.

6. Procédé selon la revendication 1, **caractérisé en ce que** le sablage de pics de contrainte est réalisé brièvement à <10s.

7. Procédé selon la revendication 1, **caractérisé en ce que** l'échauffement s'effectue à de 120...160 ° C, avant l'échauffement et après l'échauffement, des valeurs étant enregistrées selon les états de contraintes respectifs.

8. Procédé selon la revendication 1, **caractérisé en ce que** les valeurs définies de manière ciblée de contraintes en compression résiduelles (σ-_{E}) sont introduites au moyen d'une pression de projection dans des ordres de ± p1.

9. Procédé selon l'une quelconque des revendications 1 à 8, **caractérisé en ce que** dans les parties (2.2, 2.3, 2.4) sont appliquées des contraintes résiduelles en compression (σ-_{E}) de l'ordre de >-0,6 MPa ×10³ à <2,0 MPa ×10³.

10. Procédé selon l'une quelconque des revendications 1 à 9, **caractérisé en ce que** les contraintes résiduelles en compression (σ-_{E}) introduites sont contrôlées et enregistrées en tant qu'une intensité mesurée au moyen de la mesure de l'intensité d'Almen.

11. Procédé selon l'une quelconque des revendications 1 à 10, **caractérisé en ce que** la courbe de contrainte (σ) selon la mesure de l'intensité d'Almen est utilisée en tant que courbe de contrainte σ de référence ou théoriquement supposable pour des lots ou des séries de crémaillères (1) qui devront être usiné(e)s avec des paramètres identiques et selon l'invention.

12. Procédé selon l'une quelconque des revendications 1 à 11, **caractérisé en ce que** la courbe de contraintes résiduelles (σ) de contraintes en traction (σ+) et de contraintes en compression (σ-) est transformée en fonction du type d'une denture (2) iᵥₐᵣ ou iₖₒₙₛₜₐₙₜ ou de la caractéristique d'une matière de la crémaillère (1) en une courbe de contraintes résiduelles (σ_{zul}) admissibles.

13. Procédé selon l'une quelconque des revendications 1 à 12, **caractérisé en ce que** l'on optimise le dimensionnement d'un diamètre (D) de la crémaillère (1) comme une crémaillère de direction d'un véhicule à l'égard de charges par essieu identiques d'un autre véhicule.
